# FASCICULE DE BREVET EUROPEEN

(11) **EP 0 404 871 B1**
(45) Date de publication et mention de la délivrance du brevet: **22.11.2000**
(21) Numéro de dépôt: 89909780.2
(22) Date de dépôt: 30.08.1989
(51) Int. Cl.: A61B 17/225

(54) **APPAREIL DE REPERAGE ET DE VISUALISATION EN TEMPS REEL DE CONCRETION**
GERÄT ZUM ZIELEN UND VISULAISIEREN VON KONKREMENTEN IN ECHTZEIT
APPARATUS FOR LOCATING AND VISUALIZING IN REAL TIME CONCRETIONS

(30) Priorité: 31.08.1988 FR 8811414
(43) Date de publication de la demande: 02.01.1991
(73) Titulaire: TECHNOMED MEDICAL SYSTEMS, 69120 Vaulx-en-Velin (FR); INSTITUT NATIONAL DE LA SANTE ET DE LA RECHERCHE MEDICALE (INSERM), 75654 Paris Cédex 13 (FR)
(72) Inventeur: LACRUCHE, Bernard, F-69003 Lyon (FR); CATHIGNOL, Dominique, F-69740 Genas (FR); LACOSTE, François, F-69006 Lyon (FR)
(74) Mandataire: Portal, Gérard
(86) Numéro de dépôt international: FR8900432
(87) Numéro de publication internationale: WO9001904

(56) Documents cités:
- EP-A- 0 169 311
- DE-A- 3 617 032
- FR-A- 2 587 893
- FR-A- 2 608 913

## Description

La présente invention concerne essentiellement un appareil de repérage et de visualisation en temps réel de concrétion.

Les concrétions se forment naturellement dans des cavités du corps des mammifères, en particulier des êtres humains. La formation de ces concrétions entraîne rapidement une obstruction des voies naturelles conduisant inévitablement à des troubles physiologiques graves pouvant mettre en danger rapidement la vie du mammifère en question.

Depuis quelques années, divers appareils ont été proposés permettant de détruire les concrétions en fragments de faible dimension évacuables par les voies naturelles, par voie extracorporelle à l'aide de générateurs d'ondes de pression dirigées sur la concrétion. Par exemple, le brevet US RIEBER n° 2 559 227 décrit un appareil générateur d'ondes de pression comprenant un réflecteur ellipsoïdal tronqué 80 dans lequel sont générées des ondes de pression par décharge ou arc électrique entre deux électrodes concourantes au premier foyer de l'ellipsoïde, de manière à détruire une cible, par exemple formée par une concrétion, disposée au deuxième foyer de l'ellipsoïde.

Le problème qui se pose en pratique d'une manière aiguë est le positionnement correct de la cible à détruire exactement au deuxième foyer de L'ellipsoïde.

Pour ce faire, il est nécessaire de repérer et de déterminer la position exacte de la cible à détruire, par exemple une concrétion, notamment une lithiase rénale ou biliaire ("calcul").

Pour le repérage et le positionnement de telles cibles, on peut utiliser diverses sondes d'exploration, que ce soit rayons X ou le plus souvent de préférence à ultrasons, à l'aide de transducteurs ultrasoniques.

Par exemple, le document FR-A-2 502 485 décrit un appareil d'exploration à sondes pour le diagnostic par ultrasons à l'aide de deux bras articulés 18, 20. Le document FR-A-2 474 186 contient une description similaire.

Le document EP-A-0 169 311 décrit un dispositif de positionnement dans l'espace d'une sonde d'exploration 28.

Les demandeurs ont également proposé un dispositif de positionnement dans l'espace d'une sonde d'exploration dans FR-A-2 598 073.

Cependant, il s'avère qu'il est nécessaire, en pratique pour Le clinicien, de pouvoir contrôler en temps réel la position de la concrétion et de suivre sa fragmentation pendant le traitement.

Diverses solutions ont déjà été proposées pour permettre de contrôler la position de la concrétion et de suivre se fragmentation pendant le traitement.

Par exemple, le document FR-A-2 587 893 décrit un appareil de repérage des fragments d'un calcul associé à un lithotripteur. Le lithotripteur comporte une coupelle sphérique de focalisation 1 jouant le rôle de transducteur de puissance et l'appareil de repérage comprend un transducteur auxiliaire 2 soumis à un balayage sectoriel, monté au centre de la coupelle et couplé à un dispositif d'échographie 21-22-28. L'appareil comprend un générateur auxiliaire 360 d'excitation de transducteur de puissance 1 en impulsion à une cadence de quelques hertz et à une puissance réduite par rapport à celle des tirs. Le récepteur 22 du dispositif échographique est, pendant l'émission de ces impulsions à puissance réduite, relié à un tube cathodique auxiliaire 45 qui forme une image en échographie A pour repérer Les fragments de calcul.

Le document FR-A-2 591 467 décrit un enseignement équivalent.

Dans ces deux dispositifs, Le transducteur ou scanner de type A ou B est disposé coaxialement au générateur d'ondes de pression focalisées au foyer-cible repéré r ou 2 respectivement et devant être ajusté sur la concrétion à détruire K ou 12 respectivement.

De par cette position coaxiale du transducteur ou du scanner type A ou B, il est possible de visualiser en permanence la concrétion lorsque celle-ci a été mise en coïncidence avec le foyer de la coupelle du générateur d'ondes de pression.

Cependant, ces dispositifs sont limités en possibilité de repérage dans l'espace même s'ils peuvent être montés en rotation sur eux-mêmes et en translation dans l'axe de symétrie du générateur d'ondes de pression.

Or, une possibilité de déplacement dans l'espace de l'appareil de repérage et de visualisation de la concrétion apparaît nécessaire pour suivre le déplacement éventuel de la concrétion ainsi que des fragments obtenus au cours du traitement. Ceci est indispensable pour éviter d'émettre des ondes de pression qui n'atteignent pas la concrétion. L'opérateur doit donc pouvoir être en mesure de stopper immédiatement l'émission des ondes pour faire une nouvelle localisation.

Pour ce repérage et cette visualisation en temps réel de la concrétion, il s'avère nécessaire en pratique d'utiliser un transducteur ou scanner auxiliaire, différent du dispositif de localisation dans l'espace de la concrétion en vue d'une mise en coïncidence de cette concrétion avec le foyer-cible du générateur d'ondes de pression.

C'est pourquoi, dans le document EP-A-0 169 311, on propose un dispositif de repérage ultrasonique 26 disposé à l'extrémité d'une structure mécanique complexe pour le repérage dans l'espace de la concrétion pour en déterminer ses coordonnées permettant ensuite, par un système de calcul complexe, d'amener le foyer-cible 36 du générateur d'ondes de pression 6 en coïncidence avec la concrétion 34 à détruire.

Cet appareil de repérage 26, étant en quelque sorte immobilisé pour la mise en coïncidence de la concrétion avec le foyer-cible 34 du générateur d'ondes de pression 6, ne peut pas être utilisé par le praticien pour une visualisation en temps réel de la concrétion.

C'est pourquoi, on observe qu'il est proposé dans ce document l'adjonction d'un dispositif de visualisation en temps réel complémentaire formé par un transducteur ultrasonique ou scanner qui est dirigé sur le foyer-cible 36 et qui est monté déplaçable en rotation autour de l'axe longitudinal du générateur d'ondes de pression 6. Ce transducteur ultrasonique ou scanner présente une résolution supérieure au dispositif ultrasonique déplaçable 26 et permet une observation de la destruction de la concrétion 34 (voir page 13, lignes 6 à 16).

Un tel dispositif auxiliaire ultrasonique ou scanner a également été proposé dans le document EP-A-0 265 742 ; les deux dispositifs de repérage ultrasonique 33, 35 sont disposés à 90° de manière à fournir des plans d'observation disposés également à 90°. La position de ces transducteurs ultrasoniques ou scanner est également fixe par rapport au générateur.

On, comme il a été dit précédemment, il arrive relativement fréquemment que les concrétions ou leurs fragments se déplacent en cours de traitement. Ceci est particulièrement vrai pour le traitement des lithiases biliaires. En effet, les lithiases biliaires bougent beaucoup plus que les lithiases rénales, car elles ont beaucoup d'espace pour se déplacer et peuvent aisément "sortir" du foyer-cible après avoir été repoussées par les ondes de choc ou les mouvements du patient. En outre, les mouvements respiratoires du patient sont très importants au niveau de la vésicule étant donné que le foie et la vésicule sont en contact direct avec le diaphragme. Ainsi, une partie non négligeable des ondes de pression ou des ondes de choc n'atteint dans ce cas probablement pas la concrétion à cause des mouvements respiratoires.

Toutes les solutions antérieurement proposées, utilisant une position fixe du dispositif auxiliaire de repérage ultrasonique ou scanner, ou de mouvement limité axialement ou dans un plan, relativement au générateur d'ondes de pression, présentent l'inconvénient majeur d'une faible liberté de positionnement du dispositif de repérage rendant en pratique très difficile, voire impossible au moins dans certains cas, de repérer et visualiser effectivement en temps réel les concrétions, en particulier dans le cas de lithiases biliaires, au cours des traitements.

La présente invention a donc pour but de résoudre le nouveau problème technique consistant en la fourniture d'une solution permettant de contrôler et visualiser en temps réel des concrétions ou leurs fragments, quel que soit leur déplacement en cours de traitement.

La présente invention a encore pour but de résoudre le nouveau problème technique consistant en la fourniture d'une solution permettant de contrôler en temps réel la position d'une concrétion et d'en suivre la fragmentation pendant le traitement, pratiquement sans limiter la liberté des positionnements du dispositif de repérage de visualisation en temps réel de la concrétion, en permettant ainsi une recherche d'une fenêtre acoustique optimale.

La présente invention a encore pour but de résoudre le nouveau problème technique consistant en la fourniture d'une solution permettant de repérer et de visualiser en temps réel les concrétions ou leurs fragments en cours de traitement, indépendamment de leurs déplacements tout en ayant les déplacements du dispositif de repérage et de visualisation liés au générateur de manière à permettre une observation de la zone du foyer-cible, et présentant l'avantage déterminant d'être accessible de l'opérateur aisément pour être éventuellement changé rapidement pour permettre l'utilisation de dispositifs transducteurs ultrasoniques ou scanner de caractéristiques différentes.

La présente invention a encore pour but de résoudre le nouveau problème technique consistant en la fourniture d'une solution permettant de repérer, visualiser en temps réel les concrétions grâce à un appareil présentant une manoeuvrabilité extrêmement aisée, aboutissant en un gain de temps et une meilleure gestion du temps de traitement aboutissant en définitive à une amélioration globale de l'efficacité du traitement.

La présente invention a encore pour but de résoudre le nouveau problème technique consistant en la fourniture d'une solution permettant de repérer, de visualiser en temps réel des concrétions avec possibilité de synchronisation respiratoire grâce à la possibilité offerte au clinicien d'observer à l'instant où, pendant le cycle respiratoire, la concrétion revient au centre du point focal habituellement matérialisé par une croix sur l'image échographique.

Tous ces problèmes techniques sont résolus pour la première fois par la présente invention, d'une manière extrèmemement simple, fiable, peu coûteuse, utilisable à l'échelle industrielle.

Ainsi, la présente invention fournit un appareillage de repérage et de visualisation en temps réel d'une concrétion dans des cavités du corps d'un mamnifère, en particulier un âtre humain, destinée à être détruite en fragments évacuables par les voies naturelles à l'aide d'un générateur d'ondes de pression dirigé sur un foyer cible (F.C.) mise en coïncidence avec ladite concrétion, comprenant des moyens formateurs d'image capables de former une image en temps réel de ladite concrétion, ainsi que de l'observer en temps réel, et des moyens de couplage des moyens formateurs d'image avec ledit générateur d'ondes de pression, ces moyens de couplage étant prévus au voisinage du bord avant du générateur d'où les ondes de pression sont émises, et comprennent des moyens de déplacement en rotation des moyens formateurs d'image relativement audit générateur, lesdits moyens formateurs d'image présentant un axe longitudinal (X-X) passant par le foyer-cible (FC), caractérisé en ce que les moyens de déplacement des moyens de couplage sont prévus pour réaliser en outre un déplacement en translation des moyens formateurs d'image dans un plan de déplacement coïncidant avec un plan de symétrie du générateur incluant ledit foyer-cible (FC) et ledit axe longitudinal (X-X) des moyens formateur d'image; et les moyens de couplage comprennent des moyens de mesure d& la distance de déplacement en translation des moyens formateurs d'image.

Selon un mode de réalisation avantageux, les moyens de couplage comprennent des moyens de déplacement périphérique des moyens formateurs d'image depuis un point périphérique externe du générateur jusqu'à un autre point périphérique externe du générateur.

Selon un mode de réalisation particulier, les moyens de déplacement comprennent un support monté déplaçable à la périphérie externe du générateur d'ondes de pression. De préférence, ce support comprend une partie fixe et une partie rotative articulées entre elles par un axe d'articulation disposé sensiblement perpendiculairement au plan de symétrie du générateur passant sensiblement par l'axe longitudinal des moyens formateurs d'images.

Selon un mode de réalisation avantageux de l'appareil selon l'invention, les moyens formateurs d'images sont solidaires d'un élément intermédiaire monté déplaçable en translation dans un plan de symétrie du générateur.

Selon un autre mode de réalisation avantageux de l'appareil selon l'invention, les moyens formateurs d'images sont montés rotatifs relativement au support. De préférence, les moyens formateurs d'images sont montés rotatifs relativement à l'élément intermédiaire.

Selon un autre mode de réalisation avantageux de l'appareil selon l'invention, l'élément intermédiaire précité comporte une ouverture traversante dans laquelle les moyens formateurs d'images sont montés rotatifs.

Selon une variante de réalisation particulière, l'élément intermédiaire précité est monté sur le support par un système d'accouplement type rail, de préférence type monorail.

Selon une autre caractéristique particulière de l'appareil selon l'invention, le support précité comprend un épaulement monté déplaçable sur le bord supérieur du générateur.

Selon un mode de réalisation particulier, le générateur d'ondes de pression précité est un générateur d'ondes de choc comprenant un réflecteur ellipsoïdal.

Selon un autre mode de réalisation particulier, le générateur d'ondes de pression est un générateur d'ondes de choc de type ultrasonique, par exemple à coupelle sphérique.

Selon un autre mode de réalisation particulier de l'appareil selon l'invention, les moyens formateurs d'images précités comprennent au moins un transducteur ultrasonique ou scanner, de préférence de type B, à temps réel.

Selon un deuxième aspect, la présente invention fournit également un appareil de génération d'ondes de pression, caractérisé en ce qu'il comprend un appareil de repérage et de visualisation en temps réel de concrétion tel que précédemment défini. Cet appareil de génération d'ondes de pression est de préférence un lithotriteur. En particulier, ce lithotriteur est un lithotriteur de type hydraulique générant des ondes de choc hydrauliques, ou un lithotriteur de type ultrasonique générant des ondes de choc ultrasoniques.

On conçoit ainsi que l'invention permet d'apporter tous les avantages techniques déterminants précédemment énontés, les problèmes techniques précédemment énoncés.

D'autres buts, caractéristiques et avantages de l'invention apparaîtront clairement à la lumière de la description explicative qui va suivre faite en référence aux dessins annexés, représentant un mode de réalisation actuellement préféré de l'invention, donnés simplement à titre d'illustration et qui ne saurait donc en aucune façon Limiter la portée de l'invention. Dans les dessins :
- la figure 1 est une vue en demi-coupe axiale d'un appareil générateur d'ondes de pression, constitué ici par exemple d'un lithotriteur hydraulique à réflecteur ellipsoïdal du type décrit par RIEBER dans US 2 559 227 ou dans FR-A-2 240 795, équipé d'un appareil de repérage et de visualisation en temps réel selon l'invention, selon la ligne ce coupe I-I de la figure 2 :
- la figure 2 est une vue selon la ligne ce coupe II-II de la figure 1 permettant de voir le dessus et la totalité du réflecteur ellipsoïdal représenté à la figure 1 ; et
- la figure 3 est une vue en coupe selon la ligne de coupe III-III de la figure 1.

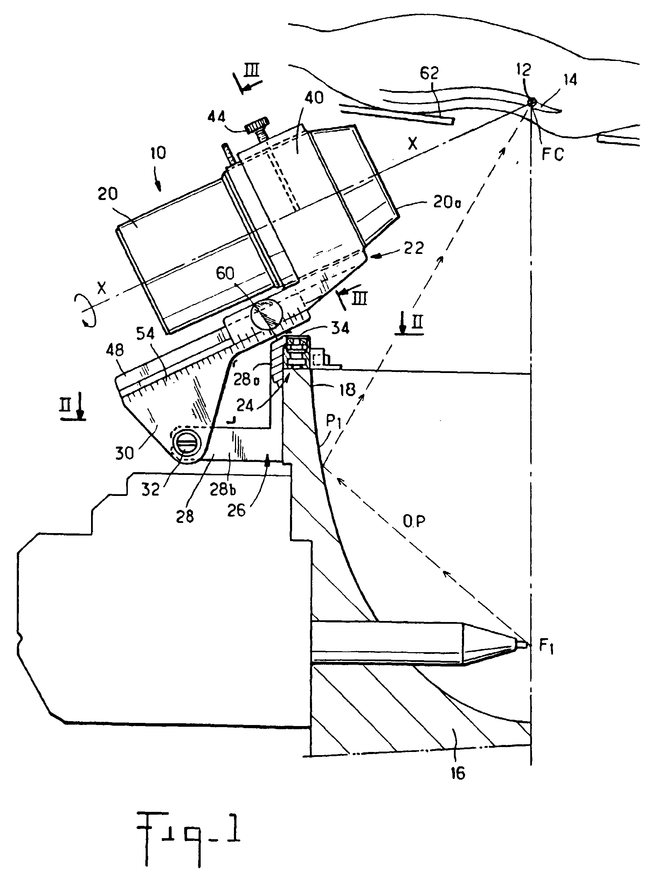

En référence aux figures 1 à 3, on a représenté un appareil selon l'invention représenté par le numéro de référence général 10, de repérage et de visualisation en temps réel d'une concrétion 12 dans des cavités 14 du corps d'un mammifère, en particulier ici un être humain, destinée à être détruite en fragments évacuables par les voies naturelles, à l'aide d'un générateur d'ondes de pression, repéré par le numéro de référence général 16. Ce générateur 16 est capable de générer des ondes de pression dirigées vers un foyer-cible FC mis en coincidence avec la concrétion 12, comme clairement visible à la figure 1.

Cet appareil 10 de repérage et de visualisation en temps réel de concrétion comprend des moyens formateurs d'images 20, capables de former une image en temps réel de la concrétion 12. De tels moyens formateurs d'images sont bien connus et sont de préférence constitués selon l'invention par un transducteur ultrasonique ou scanner, en particulier de type B encore appelé sonde à ultrasons à plan de coupe sectoriel. De tels moyens formateurs d'images sont bien connus à l'homme de L'art des,ultrasons ou de l'échographie et ne sont donc pas décrits en détail.

Cet appareil 10 comprend également des moyens repérés par le numéro de référence général 22, de couplage des moyens formateurs d'images 10 avec le générateur d'ondes de pression 16. Ces moyens de couplage 22 sont prévus vers le bord avant 18 du générateur d'ondes de pression 16 d'où les ondes de pression (O.P.) sont émises du générateur depuis le foyer d'émission F₁ comme symbolisé en trait mixte repéré O.P.

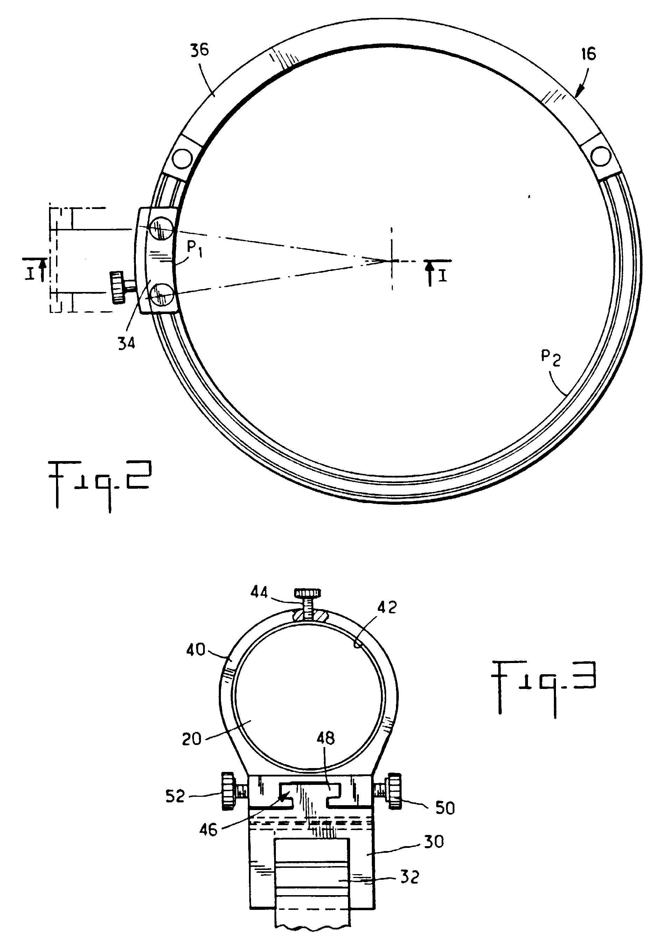

Selon la présente invention, cet appareil 10 est caractérisé en ce pue les moyens de couplage 22 comprennent des moyens repérés par le numéro de référence général 24 de déplacement des moyens formateurs d'images depuis un point périphérique externe par exemple P1 figure 2, jusqu'à un autre point périphérique externe, par exemple P2 figure 2 du générateur 16.

Selon un mode de réalisation particulier, les moyens formateurs d'images 20 sont solidaires d'un support 26 monté déplaçable à la périphérie externe du générateur d'ondes 16, comme cela est clairement visible et compréhensible à partir des figures 1 à 3.

Selon un mode de réalisation particulièrement avantageux de l'invention, le support 26 comprend une partie dite fixe 28 et une partie dite rotative 30, articulées entre elles par un, axe d'articulation 32 disposé sensiblement perpendiculairement au plan de symétrie du générateur 16 passant sensiblement par l'axe longitudinal X-X des moyens formateurs d'images 20, qui est le plan de coupe de la figure 1. Ainsi, le générateur 16 étant généralement disposé pour avoir l'axe focal F1-FC vertical, l'axe d'articulation 32 est disposé sensiblement horizontalement de sorte que la partie rotative 30 se déplace en rotation dans le plan vertical.

Selon une autre caractéristique particulière, l'élément support 26 comprend un épaulement 34, qui est naturellement ici solidaire de la partie fixe 28, monté déplaçable sur le bord supérieur 36 clairement visible à la figure 2, du générateur 16 tout en étant lié à celui-ci. Par exemple, ce déplacement est réalisé grâce à un système à roulement à billes bien connu de l'homme de l'art ou un système de rail également bien connu.

Selon un mode de réalisation préféré, le support 26 est monté déplaçable relativement au générateur 16 selon un angle de déplacement important, de préférence supérieur à 90°, encore de préférence supérieur à 360°.

Selon un mode de réalisation particulier, la partie fixe 28 du support 26 est montée déplaçable, par exemple par son épaulement 34, sur le bord supérieur 36 du générateur 16, par un système d'accouplement type rail, de préférence type monorail.

Selon un mode de réalisation particulier de l'invention, les moyens formateurs d'images 20 sont solidaires d'un élément intermédiaire 40 monté déplaçable en translation dans un plan sensiblement perpendiculaire à l'axe d'articulation 32, qui coïncide de préférence avec le plan de symétrie du générateur 16.

Selon un autre mode de réalisation particulier de l'invention, les moyens formateurs d'images 20 sont montés rotatifs relativement au support 26, et en particulier à l'élément intermédiaire 40.

Selon un mode de réalisation particulièrement avantageux, l'élément intermédiaire 40 comporte une ouverture traversante 42 dans laquelle les moyens formateurs d'images 20 sont montes rotatifs. En général, les moyens formateurs d'images 20 sont montés dans un boîtier étanche de forme sensiblement cylindrique comme représenté aux figures 1 et 3. On peut prévoir naturellement un moyen de verrouillage 44 en une position quelconque, tel qu'une vis.

Selon un autre mode de réalisation particulier de l'invention, l'élément intermédiaire 40 est monté déplaçable sur le support 28, plus précisément sur sa partie rotative 30, de préférence par un système d'accouplement 46 de type rail, de préférence type monorail 48 clairement visible à la figure 3. Ce système d'accouplement 46 à monorail 48 est bien connu en mécanique et il n'est donc pas nécessaire de le décrire plus précisément. Il présente cependant l'avantage de permettre un déplacement en translation très précis, et pouvant être verrouillé dans une position quelconque très aisément grâce à la présence du moyen de verrouillage 50, 52, tel que des vis. On peut prévoir sur au moins une face latérale apparente de la partie rotative 30 du support 28, La présence d'une réglette 54 sur laquelle est matérialisée la position des moyens formateurs d'images 20 pour laquelle Le foyer-cible FC apparaît au centre de l'image échographique. Cette réglette 54 indique de préférence également la distance séparant l'extrémité avant 20a des moyens formateurs d'images 20 du foyer-cible FC.

Selon un autre mode de réalisation particulier de l'invention, la partie fixe 28 du support 26 présente en coupe la forme d'un L adossé au générateur 16 comme clairement visible à la figure 1, ayant la barre verticale 28a du L comportant l'épaulement 34 et la barre horizontale 28b comportant vers son extrémité libre l'axe de rotation 32. De préférence, la forme de la partie rotative 30 est telle qu'avec cette disposition, la partie rotative 30 présente sa face supérieure comportant le monorail 48, en position sensiblement de repos, telle que représentée à la figure 1, reposant sur l'extrémité supérieure de la partie fixe 28, selon une direction formant un angle sensiblement égal à environ 45° par rapport à l'horizontal. En conséquence, l'axe longitudinal X-X des moyens formateurs d'images 20 sont également disposés selon un angle d'environ 45° qui constitue un angle préférentiel de fenêtre acoustique. Pour faciliter cet appui de la partie rotative 30 sur la partie fixe 28, en particulier le bord supérieur de la partie fixe 28 en L, on peut prévoir un méplat 60 à la jonction entre l'épaulement 34 et la barre verticale 28a. De préférence, l'axe de rotation 32 est prévu de telle sorte qu'il soit possible de verrouiller la partie rotative 30 dans une position verticale quelconque relativement à la partie fixe 28, grâce à un moyen de verrouillage approprié bien connu à l'homme de l'art, tel que vis (non représentée).

Il est à noter que la structure représentée aux figures 1 à 3 fait partie intégrante de l'invention et constitue donc une partie intégrante de la présente description.

Avec l'appareil de repérage et de visualisation en temps réel selon la présente invention tel que précédemment décrit, on obtient une grande liberté de positionnement des moyens formateurs d'images 20. Ceci permet de sélectionner la fenêtre acoustique optimale pour obtenir une image échographique de qualité. Ceci permet pratiquement dans tous Les cas de suivre précisément La fragmentation des concrétions. En outre, grâce à la structure de L'appareil selon L'invention, la manoeuvrabilité des moyens formateurs d'images 20 est exceptionnelle, de sorte que l'on obtient un choix de fenêtres acoustiques pratiquement illimité, ce qui permet d'utiliser les performances des moyens formateurs d'images, tels qu'un scanner type B ou échographe, et garantit une facilité d'utilisation optimale.

Il est en outre extrêmement aisé de changer les moyens formateurs d'images 20 de manière à prendre d'autres moyens formateurs d'images ayant par exemple des fréquences d'émission différentes pour permettre de repérer plus facilement des concrétions trop profondes ou trop superficielles.

En outre, il est également extrêmement aisé selon l'invention de visualiser La zone de passage des ondes de pression émises par le générateur d'ondes de pression 16. Il est possible de visualiser l'ensemble de la zone de passage en faisant tourner les moyens formateurs d'images 20 autour de l'axe longitudinal X-X.

Grâce à l'appareil selon l'invention, l'opérateur reçoit une information constante sur le processus de fragmentation. Si la concrétion quitte la zone focale FC, l'opérateur est immédiatement informé. Il sait alors que les ondes de pression ultérieurement émises sont inefficaces puisqu'elles n'atteindront plus la concrétion.

En interrompant l'émission des ondes de pression et en repositionnant la concrétion sur le point focal, il s'assure que l'ensemble des ondes de pression émises contribue à la fragmentation de la concrétion.

De ce fait, le nombre moyen d'ondes de pression par traitement est théoriquement réduit puisqu'on élimine les coûts inutiles. On aboutit ainsi à un gain de temps et à une meilleure gestion du temps de traitement. La relocalisation de la concrétion intervient toujours au moment opportun.

L'invention offre également la possibilité d'une synchronisation respiratoire.

Il est connu depuis toujours que les mouvements respiratoires provoquent des déplacements importants des concrétions. Ainsi, si l'opérateur le juge nécessaire, grâce à la visualisation en temps réel assurée en toute circonstance, l'opérateur pourra commander l'émission d'une onde de pression lorsque, pendant le cycle respiratoire, la concrétion revient sur le centre du point focal FC, qui est matérialisé habituellement par une croix sur l'image échographique.

Grâce à l'invention, il est également possible de positionner les moyens formateurs d'images 20 en contact contre le corps du patient, ce qui permet d'améliorer la résolution (il est à noter que ta position de la table 62 de support du patient est volontairement incorrecte pour faciliter la compréhension du dessin avec le corps du patient qui n'est pas à l'échelle ; habituellement, cette position est nettement en retrait, de sorte qu'aucun obstacle ne peut gêner le déplacement des moyens 10, notamment selon X-X ou dans le plan vertical.

L'appareil selon l'invention peut être monté sur tout type d'appareil générateur d'ondes de pression, qu'il s'agisse de générateur pour la lithotrypsie hydraulique, en particulier lorsqu'il s'agit de réflecteur ellipsoïdal comme représenté aux figures 1 et 2, ou dans le cas de générateur ultrasonique, par exemple comprenant une calotte sphérique pourvue d'une mosaïque de transducteurs piézo-électriques.

Pour faciliter la mise en rotation des moyens formateurs d'images 20 relativement à l'élément intermédiaire 40, il est par exemple possible d'adopter la structure décrite et représentée en référence à la figure 5 de FR-A-2 598 073 des demandeurs.

On comprend ainsi que de nombreuses variantes et modifications de l'appareil selon l'invention sont possibles sans sortir de son cadre.

## Revendications

1. Appareil (10) de repérage et de visualisation en temps réel d'une concrétion (12) dans des cavités (14) du corps d'un mamnifère, en particulier un être humain, destinée à être détruite en fragments évacuables par les voies naturelles à l'aide d'un générateur (16) d'ondes de pression dirigé sur un foyer cible (F.C.) mis en coïncidence avec ladite concrétion (12), comprenant des moyens formateurs d'image (20) capables dc former une image en temps réel de ladite concrétion (12), ainsi que de l'observer en temps réel, et des moyens (22) de couplage des moyens formateurs d'image (20) avec ledit générateur (16) d'ondes de pression, ces moyens de couplage (22) étant prévus au voisinage du bord avant (18) du générateur (16) d'où les ondes de pression sont émises, et comprennent des moyens de déplacement en rotation (24) des moyens formateurs d'image (20) relativement audit générateur (16), lesdits moyens formateurs d'image (20) présentant un axe longitudinal (X-X) passant par le foyer-cible (FC),
caractérisé en ce que les moyens de déplacement des moyens de couplage (22) sont prévus pour réaliser en outre un déplacement en translation des moyens formateurs d'image (20) dans un plan de déplacement coïncidant avec un plan de symétrie du générateur incluant ledit foyer-cible (FC) et ledit axe longitudinal (X-X) des moyens formateur d'image (20); et les moyens de couplage (22) comprennent des moyens (54) de mesure de la distance de déplacement en translation des moyens formateurs d'image (20).

2. Appareil selon la revendication 1, caractérisé en ce que les moyens de couplage (22) comprennent un support (26) solidarisant les moyens formateurs d'image (20) du générateur (16) d'ondes de pression.

3. Appareil selon la revendication 2, caractérisé en ce que les moyens de couplage (22) comprennent des moyens (24) de déplacement périphérique des moyens formateurs d'image (20) depuis un point périphérique externe (P₁) du générateur (16) jusqu'à un autre point périphérique externe (P₂) du générateur (16) ; les moyens formateurs d'image (20) étant solidaires du support (26) monté déplaçable à la périphérie externe du générateur (16).

4. Appareil selon la revendication 2 ou 3, caractérisé en ce que le support (26) précité comprend une partie fixe (28) et une partie rotative (30) articulées entre elles par un axe d'articulation (32) disposé sensiblement perpendiculairement au plan de symétrie du générateur (16) passant par le foyer cible (FC) et par l'axe longitudinal (X-X) des moyens formateurs d'image (20).

5. Appareil selon l'une des revendications 1 à 4, caractérisé en ce que les moyens de couplage (22) comprennent un élément intermédiaire (40) monté déplaçable en translation selon l'axe longitudinal (X-X) des moyens formateurs d'image (20).

6. Appareil selon l'une des revendications 3 à 5, caractérisé en ce que les moyens formateurs d'image (20) sont montés rotatifs autour de l'axe longitudinal (X-X).

7. Appareil selon l'une des revendications 1 à 6, caractérisé en ce que les moyens de couplage (22) comprennent un élément intermédiaire (40) comportant une ouverture traversante (42) dans laquelle les moyens formateurs d'image sont montés rotatifs relativement à l'élément intermédiaire (40), permettant ainsi un déplacement en translation des moyens formateurs d'image (20) en direction du foyer-cible (FC) et un déplacement en rotation autour de l'axe longitudinal (X-X).

8. Appareil selon l'une des revendications 3 à 7, caractérisé en ce que l'élément intermédiaire (40) est monté sur le support (26) par un système d'accouplement (46) type rail, de préférence type monorail (48).

9. Appareil selon l'une des revendications 2 à 8, caractérisé en ce que le support (26) comprend un épaulement (34) monté déplaçable sur le bord supérieur (36) du générateur (16).

10. Appareil selon l'une des revendications 4 à 9, caractérisé en ce que la partie fixe (28) du support (26) présente en coupe une forme en L adossé au générateur (16), de préférence une extrémité du L étant montée déplaçable sur le bord supérieur (36) du générateur (16) et l'autre extrémité comportant l'axe d'articulation (32) de ladite partie fixe (28) relativement à la partie rotative (30).

11. Appareil selon l'une des revendications 1 à 10, caractérisé en ce que les moyens de mesure (54) de la distance de déplacement en translation des moyens formateurs d'image (20) matérialisent la position des moyens formateurs d'image (20) pour laquelle Je foyer-cible (FC) apparaît au centre de l'image échographique, ou permettent d'indiquer de préférence également la distance séparant l'extrémité avant (20a) des moyens formateurs d'image (20) du foyer-cible (FC), ces moyens de mesure (54) pouvant être par exemple une réglette.

12. Appareil selon l'une des revendications 1 à 11, caractérisé en ce que, en position habituelle, l'axe (X-X) longitudinal des moyens formateurs d'image (20) forme un angle d'environ 45° par rapport à l'horizontale.

13. Appareil générateur d'ondes de pression pour la destruction de cibles telles que des concrétions, notamment des lithiases rénales, biliaires, caractérisé en ce qu'il comprend au moins un appareil de repérage et de visualisation en temps réel tel que défini selon l'une quelconque des revendications 1 à 12.

14. Appareil générateur selon la revendication 13, caractérisé en ce qu'il comprend un réflecteur ellipsoïdal, les moyens formateurs d'images précités étant de préférence un transducteur ultrasonique, en particulier un scanner type B.

## Claims

1. An apparatus (10) for real-time tracking and imaging of a concretion (12) in a cavity (14) of the body of a mammal, and in particular a human being, the concretion being destined to be destroyed into fragments that can be evacuated by natural passages using a pressure wave generator (16) directed towards a target-focus (F.C.) put into coincidence with said concretion (12), comprising image-forming means (20) capable of forming a real-time image of said concretion (12) for real-time observation of the latter, and coupling means (22) for coupling said image-forming means (20) with said pressure wave generator (16), said coupling means (22) being provided in the vicinity of the front edge (18) of the generator (16) from which pressure waves are emitted and comprise means (24) for displacing in rotation image-forming means (20) with respect to said generator (16), said image-forming means (20) having a longitudinal axis (X-X) passing through the target-focus (FC),
characterised in that the means for displacing the coupling means (22) are provided for the purpose of carrying out in addition a displacement in translation of the image-forming means (20) in a plane of displacement which coincides with a symmetry plane of the generator including said target-focus (FC) and said longitudinal axis (X-X) of the image-forming means (20); and the coupling means (22) comprise means (54) for measuring the distance of displacement in translation of the image-forming means (20).

2. The apparatus according to claim 1, characterised in that the coupling means (22) comprise a support (26) which is unitary with the image-forming means (20) of the pressure wave generator (16).

3. The apparatus according to claim 2, characterised in that the coupling means (22) comprise peripheral displacement means (24) for displacing image-forming means (20) from an external peripheral point (P₁) of the generator (16) to another external peripheral point (P₂) of the generator (16); the image-forming means (20) being unitary with the support (26) displaceably mounted at the external periphery of the generator (16).

4. The apparatus according to claim 2 or 3, characterised in that the aforesaid support (26) comprises a fixed part (28) and a rotatable part (30) mutually articulated by an articulation axis (32) located substantially perpendicular to the symmetry plane of the generator (16) passing through the target-focus (FC) and through the longitudinal axis (X-X) of the image-forming means (20).

5. The apparatus according to one of claims 1 to 4, characterised in that the coupling means (22) comprise an intermediate element (40) displaceably mounted in translation along the longitudinal axis (X-X) of the image-forming means (20).

6. The apparatus according to one of claims 3 to 5, characterised in that the image-forming means (20) are rotatably mounted about the longitudinal axis (X-X).

7. The apparatus according to one of claims 1 to 6, characterised in that the coupling means (22) comprise an intermediate element (40) having a through opening (42) inside which the image-forming means are rotatably mounted with respect to the intermediate element (40), thus allowing a displacement in translation of the image-forming means (20) in the direction of the target-focus (FC) and a displacement in rotation about the longitudinal axis (X-X).

8. The apparatus according to one of claims 3 to 7, characterised in that the intermediate element (40) is mounted on the support (26) by a rail type coupling system (46), preferably of the monorail type (48).

9. The apparatus according to one of claims 2 to 8, characterised in that the support (26) has a shoulder (34) displaceably mounted on the upper edge (36) of the generator (16).

10. The apparatus according to one of claims 4 to 9, characterised in that the fixed part (28) of the support (26) has a cross-section in the shape of an L abutting the generator (16), preferably one end of the L being mounted displaceable on the upper edge (36) of the generator (16) and the other end including the articulation axle (32) of said fixed part (28) with respect to the rotatable part (30).

11. The apparatus according to one of claims 1 to 10, characterised in that the means (54) for measuring the displacement distance in translation of the image-forming means (20) materialise the position of the image-forming means (20) for which the target-focus (FC) appears at the center of the echograph image, or preferably also enable to indicate the distance separating the front end (20a) of the image-forming means (20) from the target-focus (FC), such measuring means (54) can be a rule, for example.

12. The apparatus according to one of claims 1 to 11, characterised in that, in a usual working position, the longitudinal axis (X-X) of the image-forming means (20) form an angle of approximately 45° with respect to the horizontal.

13. A pressure wave generating apparatus for destroying targets such as concretions, and in particular kidney stones and biliary concretions, characterised in that it comprises at least one real-time tracking and imaging apparatus as defined according to any one of claims 1 to 12.

14. The generating apparatus according to claim 13, characterised in that it comprises an ellipsoidal reflector, the aforesaid image-forming means being preferably an ultrasonic transducer, in particular a B-type scanner.

## Patentansprüche

1. Vorrichtung (10) zur Echtzeit-Ortung und -Visualisierung eines Konkrements (12) in Körperhohlräumen von Säugetieren, insbesondere von Menschen, das dazu bestimmt ist, in über natürliche Wege entfernbare Teile unter Zuhilfenahme eines Druckwellengenerators (16) zerkleinert zu werden, welcher auf einem Zielfokus (F.C.), der mit dem festen Teil (12) zusammenfällt, gerichtet wird, mit Bildformationsmittel (20), die zur Formung eines Bildes eines Konkrements (12) in Echtzeit sowie zu dessen Beobachtung in Echtzeit geeignet sind, und mit Mitteln (22) zum Koppeln der Bildformationsmittel (20) mit dem Druckwellengenerator (16), wobei diese Kopplungsmittel (22) in der Nähe des vorderen Randes (18) des Generators (16) vorgesehen sind, von wo die Druckwellen gesendet werden, und Mittel (24) zum drehenden Verstellen der Bildformationsmittel (20) relativ zum Generator (16) aufweisen, wobei die Bildformationsmittel (20) eine durch den Zielfokus (FC) verlaufende Längsachse (X-X) aufweisen,
dadurch gekennzeichnet, dass die Verstellmittel der Kopplungsmittel (22) dazu vorgesehen sind, außerdem eine translatorische Verstellung der Bildformationsmittel (20) in einer Verstellebene zu bewirken, die mit einer Symmetrieebene des Generators zusammenfällt, die den Zielfokus (FC) und die Längsachse (X-X) der Bildformationsmittel (20), enthält; und die Kopplungsmittel (22) Mittel (54) zum Messen der Strecke der translatorischen Verstellung der Bildformationsmittel aufweisen.

2. Vorrichtung nach Anspruch 1, dadurch gekennzeichnet, dass die Kopplungsmittel (22) einen Träger (26) aufweisen, welcher die Bildformationsmittel (20) mit dem Druckwellengenerator (16) fest verbindet.

3. Vorrichtung nach Anspruch 2, dadurch gekennzeichnet, dass die Kopplungsmittel (22) Mittel (24) zur umfangsmäßigen Verstellung der Bildformationsmittel (20) von einem Außenumfangspunkt (P1) des Generators (16) zu einem anderen Außenumfangspunkt (P2) des Generators (16) aufweisen; wobei die Bildformationsmittel (20), fest mit dem Träger (26) verbunden sind, der am Außenumfangspunkt des Generators (16) verstellbar angebracht ist.

4. Vorrichtung nach Anspruch 2 oder 3, dadurch gekennzeichnet, dass der Träger (26) einen testen Teil (28) und einen drehbaren Teil (30) aufweist, welche schwenkbar miteinander über eine Gelenkachse (32) verbunden sind, die im Wesentlichen senkrecht zur Symmetrieebene des Generators (16) angeordnet ist, welche durch den Zielfokus (FC) und die Längsachse (X-X) der Bildformationsmittel (20) verläuft.

5. Vorrichtung nach einem der Ansprüche 1 bis 4, dadurch gekennzeichnet, dass die Kopplungmittel (22) ein Zwischenelement (40) aufweisen, welches translatorisch gemäß mit der Längsachse (X-X) der Bildformationsmittel (20) verstellbar angebracht ist.

6. Vorrichtung nach einem der Ansprüche 3 bis 5, dadurch gekennzeichnet, dass die Bildformationsmittel (20) um die Längsachse (X-X) drehbar angebracht sind.

7. Vorrichtung nach einem der Ansprüche 1 bis 6, dadurch gekennzeichnet, dass die Kopplungsmittel (22) ein Zwischenelement (40) aufweisen, welches von einer Öffnung (42) durchquert ist, in welcher die Bildformationsmittel relativ zum Zwischenelement (40) drehbar angebracht sind, wobei auf diese Weise eine translatorische Verstellbewegung der Bildformationsmittel (20) in Richtung Zielfokus (FC) und eine Drehbewegung um die Längsachse (X-X) erlaubt wird.

8. Vorrichtung nach einem der Ansprüche 3 bis 7, dadurch gekennzeichnet, dass das Zwischenelement (40) mittels eines Kupplungssystems (46) vom Schienentyp, vorzugsweise vom Einschienentyp (48), am Träger (26) angebracht ist.

9. Vorrichtung nach einem der Ansprüche 2 bis 8, dadurch gekennzeichnet, dass der Träger (26) eine Schulter (34) aufweist, welche am oberen Rand (36) des Generators (16) verstellbar angebracht ist.

10. Vorrichtung nach einem der Ansprüche 4 bis 9, dadurch gekennzeichnet, dass der feste Teil (28) des Trägers (26) im Schnitt die Form eines an den Generator (16) angelehnten L aufweist, wobei vozugsweise ein Schenkel des L am oberen Rand (36) des Generators (16) verstellbar angebracht ist, und der andere Schenkel die Gelenkachse (32) zwischen dem festen Teil (28) mit dem drehbaren Teil (30) enthält.

11. Vorrichtung nach einem der Ansprüche 1 bis 10, dadurch gekennzeichnet, dass die Mittel (54) zur Messung der Strecke der translatorischen Verstellung der Bildformationsmittel (20) die Position der Bildformationsmittel (20) markieren, für welche der Zielfokus (FC) im Zentrum des echographischen Bildes erscheint, oder, vorzugsweise auch die Anzeige der Strecke erlauben, welche das vordere Ende (20a) der Bildformationsmittel (20) vom Zielfokus (FC) beabstandet, wobei diese Mittel (54) beispielsweise ein Lineal sein können.

12. Vorrichtung nach einem der Ansprüche 1 bis 11, dadurch gekennzeichnet, dass in üblicher Position die Längsachse (X-X) der Bildformationsmittel (20) einen Winkel von ungefähr 45° zur Horizontalen bildet.

13. Druckwellengeneratorvorrichtung zur Zerstörung von Zielen, wie von Konkrementen, insbesondere von Nierensteinen, Gallensteinen, dadurch gekennzeichnet, dass sie mindestens eine Vorrichtung zur Ortung und Visualisierung in Echtzeit nach einem der Ansprüche 1 bis 12 aufweist.

14. Generatorvorrichtung nach Anspruch 13, dadurch gekennzeichnet, dass sie einen ellipsoidischen Reflektor aufweist, wobei die Bildformationsmittel vorzugsweise ein Ultraschallwandler, insbesondere ein Typ-B-Scanner, sind.
